# EUROPÄISCHE PATENTANMELDUNG

(11) **EP 0 528 205 A1**
(43) Veröffentlichungstag der Anmeldung: **24.02.1993**
(21) Anmeldenummer: 92112669.4
(22) Anmeldetag: 24.07.1992
(51) Int. Cl.: A61M 39/00

(54) **Einführungsschleuse für einen Katheter**

(30) Priorität: 21.08.1991 DE 4127628
(71) Anmelder: VYGON GMBH & CO KG, D-52070 Aachen (DE)
(72) Erfinder: Heiliger, Raymund, Dr., W-5120 Herzogenrath (DE)
(74) Vertreter: Bauer, Hubert, Dipl.-Ing.

(57) **Zusammenfassung**

Um durch eine in Abwesenheit ebenso wie in Anwesenheit eines Katheters dichte Einführungsschleuse einen Katheter einzubringen, ist die Einführungsschleuse mit einem elastischen Ventilkörper (1) ausgestattet. In diesem ist durch Y-förmig zueinander angeordnete Schlitze (3) eine selbsttätig verschließbare Durchtrittsöffnung gebildet, die für die Katheterspitze dadurch besonders leicht auffindbar ist, daß der Ventilkörper (1) auf seiner proximalen Seite mit einer kegelstumpfförmigen Aussparung (2) versehen ist. Durch die Aussparung (2) wird zudem die Flexibilität der durch die Schlitze (3) begrenzten Materialzwickel gesteigert, welche sich in Anwesenheit des Katheters formschlüssig gegen dessen Außenseite abdichtend anlegen.

## Beschreibung

Die Erfindung betrifft eine Einführungsschleuse für einen Katheter mit einem elastischen Ventilkörper, der eine selbsttätig verschließbare Durchtrittsöffnung für den Katheter aufweist und diesen dicht umschließt. Dabei ist die Durchtrittsöffnung durch Schlitze gebildet, die von der Mittelachse des kreisscheibenförmigen Ventilkörpers aus radial nach außen gerichtet sind und die sich im wesentlichen axial durch den Ventilkörper erstrecken.

Eine aus der US 35 85 996 bekannte Einführungsschleuse besteht aus einer großen Anzahl von Einzelteilen, was hinsichtlich der Fertigung, Handhabung und Wartung nachteilig ist. Zudem erfolgt bei dieser bekannten Einführungsschleuse die Abdichtung nicht unmittelbar am Katheter, sondern an einem besonderen Verbindungsstück, wobei eine mit einem Y-förmigen Schlitz versehene zweite Dichtung mit einer ersten Dichtung nicht so zusammenwirken kann, daß dadurch bei abwesendem Katheter eine sichere Abdichtung gegen den Blutdruck gewährleistet ist.

Aus der DE 26 23 511 C2 ist eine Einführungsschleuse der vorbeschriebenen Art bekannt, welche bei verminderter Anzahl von Einzelteilen eine rasche und zuverlässige Abdichtung einerseits direkt an einem eingeführten Katheter und andererseits bei abwesendem Katheter gegen den Blutdruck ermöglichen soll. Diese bekannte Einführungsschleuse ist gleichfalls mit zwei nebeneinander angeordneten Dichtungen ausgestattet. Die erste, aus elastischem Material gefertigte Dichtung ist mit einer Öffnung zur Einführung des Katheters versehen und weist einen etwas kleineren Durchmesser auf als der Katheter, so daß die Dichtung bei eingeführtem Katheter abdichtend an diesem anliegt. Die zweite, gleichfalls aus elastischem Material gefertigte Dichtung weist einen Y-förmigen Schlitz auf, der sich bei abwesendem Katheter gegen die benachbart angeordnete erste Dichtung abstützen soll. Bei dieser Einführungsschleuse ist trotz der zweiten Dichtung eine Leckage nur zu vermeiden, wenn die in der ersten Dichtung vorgesehene zentrische Öffnung hinreichend genau dem Katheter angepaßt ist. Sollen dagegen Katheter mit unterschiedlichen Durchmessern zum Einsatz kommen, ist mit dieser Einführungsschleuse eine stets wirksame Abdichtung nicht zu erzielen.

Schließlich ist aus der DE 34 01 440 C2 eine Einführungsschleuse der eingangs beschriebenen Art bekannt, die auch bei Kathetern mit stark unterschiedlichen Außendurchmessern eine flüssigkeitsdichte Abdichtung gewährleisten soll. Dazu ist der einstückige, aus elastischem Material hergestellte Ventilkörper mit einem ersten, sich nur zu einer ersten Stirnseite hin öffnenden Schlitz und mit einem zweiten, sich nur zu einer zweiten Stirnseite hin öffnenden Schlitz versehen. Die beiden Schlitze erstrecken sich also in axialer Richtung jeweils nur über einen Teilbereich des Ventilkörpers. Sie kreuzen sich innerhalb des Ventilkörpers und schneiden einander an von den Stirnseiten des Ventilkörpers abgesetzten bzw. zwischen diesen liegenden Punkten. Ein wesentlicher Nachteil dieser Einführungsschleuse ist darin zu sehen, daß die Ausbildung des Ventilkörpers die Einführung eines Katheters erheblich erschwert. Verfehlt die Spitze des einzuführenden Katheters den Kreuzungspunkt der Schlitze im Ventilkörper oder schließt infolge einer geringfügigen Neigung die Mittelachse des Katheters mit der Mittelachse der Einführungsschleuse einen nur geringen Winkel ein, so trifft die Katheterspitze auf das zwar elastische Material des Ventilkörpers, läßt sich aber nicht ordnungsgemäß einführen. Durch wiederholte nicht zum Ziel führende Einführungsversuche mit dem Katheter kann sogar der Ventilkörper beschädigt werden und seine Funktionsfähigkeit verlieren.

Der Erfindung liegt die Aufgabe zugrunde, eine Einführungsschleuse der eingangs beschriebenen Art so auszubilden, daß sie unter Beibehaltung des Vorteils, auch beim Einsatz von im Außendurchmesser stark unterschiedlichen Kathetern und in deren Abwesenheit einen flüssigkeitsdichten Verschluß zu gewährleisten, zudem die ordnungsgemäße Kathetereinführung wesentlich erleichtert und diese selbst dann ermöglicht, wenn die Katheterspitze nicht koaxial durch die Einführungsschleuse geführt wird.

Zur Lösung dieser Aufgabe wird von einer Einführungsschleuse der im Oberbegriff des Anspruchs 1 genannten gattungsgemäßen Art ausgegangen, welche erfindungsgemäß die in seinem kennzeichnenden Teil angegebenen Merkmale aufweist.

Durch die erfindungsgemäße Aussparung nach Art eines Trichters im Ventilkörper genügt es zur ordnungsgemäßen Einführung des Katheters, wenn die Katheterspitze zunächst innerhalb des Grundkreises der Aussparung auf den Ventilkörper auftrifft und sodann durch die schräge seitliche Begrenzungswand der Aussparung auf den durch den Deckkreis definierten Boden der Aussparung geleitet wird. Dort setzt das geschlitzte elastische Ventilkörpermaterial der Katheterspitze einen nur geringen Widerstand entgegen, so daß eine am Katheter ausgeübte geringe axiale Vorschubkraft ausreicht, den Katheter durch die Einführungsschleuse vorzuschieben.

Zudem entstehen im geschlitzten Ventilkörperbereich durch die Aussparung gegenüber der Ventilkörperdicke in der Wandstärke erheblich reduzierte Materialzwickel in Form von Kreissegmenten mit einer entsprechend erhöhten Flexibilität. Die Materialzwickel legen sich daher formschlüssig an den Außenumfang des Katheters an und begünstigen die Dichtwirkung außerordentlich.

Nach einer Ausgestaltung der Erfindung ist die radiale Erstreckung der Schlitze kleiner als der Radius des Grundkreises und größer als der Radius des Deckkreises der Aussparung.

Durch diese Ausgestaltung sind die Materialzwickel über einen entsprechend vergrößerten Anschlußquerschnitt mit dem übrigen Ventilkörper verbunden. Dies bewirkt eine Erhöhung der Rückstellkraft der Materialzwickel in ihre Ausgangsposition, sobald der Katheter aus der Einführungsschleuse entfernt ist.

Die Flexibilität der Materialzwickel läßt sich noch dadurch steigern, daß gemäß einer weiteren Ausgestaltung der Erfindung von beiden Stirnseiten des Ventilkörpers je eine kegelstumpfförmige Aussparung ausgeht.

Auch bei dieser Ausgestaltung kann der vergrößerte Anschlußquerschnitt der Materialzwickel am übrigen Ventilkörper beibehalten werden, zumal dessen Größe im wesentlichen durch die radiale Erstreckung der Schlitze wesentlich beeinflußt werden kann.

Schließlich sieht eine Ausgestaltung der Erfindung noch vor, daß die Summe der Parallelabstände zwischen dem Grundkreis und dem Deckkreis beider kegelstumpfförmigen Aussparungen kleiner ist als die Höhe des Ventilkörpers.

Durch diese Ausgestaltung ist sichergestellt, daß die Materialzwickel infolge der Aussparungen im Bereich der Projektion der Deckkreise nicht gänzlich in Fortfall kommen, sondern die Einführungsschleuse in Abwesenheit eines Katheters durch die Materialzwickel ebenso dicht geschlossen bleibt wie in Anwesenheit des Katheters.

In der Zeichnung sind zwei Ausführungsbeispiele eines Ventilkörpers einer erfindungsgemäßen Einführungsschleuse in einem vergrößerten Maßstab dargestellt. Es zeigt:

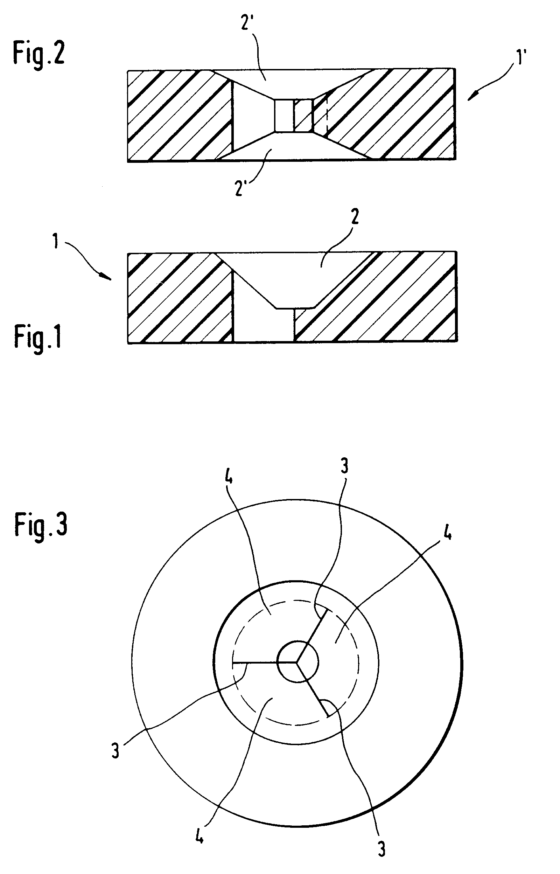

- Fig. 1: einen Ventilkörper mit einer Aussparung im Querschnitt;
- Fig. 2: einen Ventilkörper mit zwei Aussparungen im Querschnitt;
- Fig. 3: eine Draufsicht auf den Ventilkörper gemäß Fig. 1 bzw. Fig. 2.

Im nicht dargestellten Gehäuse einer Einführungsschleuse ist ein kreisscheibenförmiger Ventilkörper 1 bzw. 1' aus elastischem Material eingesetzt.

Der Ventilkörper 1 ist auf seiner proximalen Seite mit einer kegelstumpfförmigen Aussparung 2 versehen, deren Deckkreis etwa auf der Hälfte der Höhe des Ventilkörpers 1 liegt.

Der Ventilkörper 1' ist auf seinen beiden Stirnseiten mit je einer kegelstumpfförmigen Aussparung 2' versehen, deren jeweiliger Deckkreis etwa auf einem Drittel der Höhe des Ventilkörpers 1' liegt.

Sich radial von der Mittelachse des Ventilkörpers 1 bzw. 1' erstreckende, Y-förmig zueinander angeordnete Schlitze 3 erzeugen segmentförmige Materialzwickel 4, die sich infolge der Elastizität des Ventilkörpers 1 durch einen in die Schleuse eingeführten Katheter zwar verdrängen lassen, sich jedoch abdichtend gegen die Außenseite des Katheters abstützen und, sowie der Katheter aus der Einführungsschleuse entfernt ist, die in der Zeichnung dargestellte Ausgangsposition wieder einnehmen und gleichfalls die Einführungsschleuse abdichten.

## Patentansprüche

1. Einführungsschleuse für einen Katheter, mit einem elastischen Ventilkörper, der eine selbsttätig verschließbare Durchtrittsöffnung für den Katheter aufweist und diesen dicht umschließt, wobei die Durchtrittsöffnung durch Schlitze gebildet ist, die von der Mittelachse des kreisscheibenförmigen Ventilkörpers aus radial nach außen gerichtet sind und die sich im wesentlichen axial durch den Ventilkörper erstrecken, dadurch gekennzeichnet, daß der Ventilkörper (1) mindestens eine konzentrisch angeordnete, kegelstumpfförmige Aussparung (2) aufweist, deren Grundkreis auf der proximalen Stirnseite des Ventilkörpers (1) und deren Deckkreis etwa auf dessen halber Höhe liegt.

2. Einführungsschleuse nach Anspruch 1, dadurch gekennzeichnet, daß die radiale Erstreckung der Schlitze (3) kleiner als der Radius des Grundkreises und größer als der Radius des Deckkreises der Aussparung (2) ist.

3. Einführungsschleuse nach Anspruch 1 oder 2, dadurch gekennzeichnet, daß von beiden Stirnseiten des Ventilkörpers (1) je eine kegelstumpfförmige Aussparung (2') ausgeht.

4. Einführungsschleuse nach Anspruch 3, dadurch gekennzeichnet, daß die Summe der Parallelabstände zwischen dem Grundkreis und dem Deckkreis beider kegelstumpfförmigen Aussparungen (2') kleiner ist als die Höhe des Ventilkörpers (1).
